(19) Europäisches Patentamt
European Patent Office
Office européen des brevets

(11) **EP 4 693 198 A1**

(12) **EUROPEAN PATENT APPLICATION**

(43) Date of publication:
**11.02.2026 Bulletin 2026/07**

(51) International Patent Classification (IPC):
**G06T 11/00** (2026.01)

(21) Application number: **25193733.0**

(52) Cooperative Patent Classification (CPC):
**G06T 11/005; G06T 2211/408; G06T 2211/448**

(22) Date of filing: **04.08.2025**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **02.08.2024 CN 202411061950**

(71) Applicant: **Shanghai United Imaging Healthcare
Co., Ltd.
Shanghai 201807 (CN)**

(72) Inventors:
• **MA, Jinglu
Shanghai, 201807 (CN)**
• **CAO, Wenjing
Shanghai, 201807 (CN)**

(74) Representative: **Murgitroyd & Company
165-169 Scotland Street
Glasgow G5 8PL (GB)**

(54) **METHODS AND SYSTEMS FOR IMAGE RECONSTRUCTION**

(57) Provided are a method and a system for image reconstruction. The method includes: obtaining initial scanning data of a target object; performing dimension downsampling on the initial scanning data to obtain intermediate scanning data; wherein the dimension downsampling is configured to reduce the dimension of an image by merging the pixel values of multiple pixels of the image; extracting low-frequency information based on the intermediate scanning data to obtain intermediate low-frequency information corresponding to the intermediate scanning data; wherein the low-frequency information includes low-frequency images or low-frequency data; reconstructing a target reconstruction image of the target object based on the intermediate low-frequency information and the initial scanning data. In this method, the statistics of the detector units after data merging are increased by dimensionally downsampling the projection domain data collected by the detector; It does not rely on external information such as interpolation predictions or model predictions to ensure the accuracy of subsequent corrections and reconstructions, thereby improving the correction effect of the unevenness in the image and enhancing the uniformity of the image.

FIG. 7

## Description

### CROSS-REFERENCE TO RELATED APPLICATIONS

This application claims priority for a Chinese patent application No. 202411061950.5, filed on August 2, 2024, the entire contents of which are hereby incorporated by reference.

### TECHNICAL FIELD

The present disclosure relates to the field of medical imaging, and in particular, to a method and a system for image reconstruction.

### BACKGROUND

During Computed Tomography (CT) scanning procedure, X-rays are attenuated to varying degrees as they pass through different substances, and when the attenuation is large, the signal that the detector can receive will be very small, even close to zero; For such signals, it is highly likely to cause deviations in the subsequent correction and reconstruction algorithms, resulting in low-frequency ring artifacts in the reconstructed images and affecting the uniformity of the CT reconstructed images.

Traditionally, methods for correcting the uniformity of CT reconstructed images include: predicting and correcting this part of the signal using adjacent channel interpolation during the image reconstruction process, or predicting and correcting the signal based on external reference information or physical models, so that when image reconstruction is based on the corrected signal, the uniformity of the reconstructed image can be improved.

However, the results of the uniformity of CT reconstructed images obtained by using traditional methods for correcting image uniformity are still poor.

### SUMMARY

One or more embodiments of the present disclosure provide a method for image reconstruction. The method comprises: obtaining initial scanning data of a target object; performing dimension downsampling on the initial scanning data to obtain intermediate scanning data; wherein the dimension downsampling is configured to reduce the dimension of an image by merging the pixel values of multiple pixels of the image; extracting low-frequency information based on the intermediate scanning data to obtain intermediate low-frequency information corresponding to the intermediate scanning data; wherein the low-frequency information includes low-frequency images or low-frequency data;

reconstructing a target reconstruction image of the target object based on the intermediate low-frequency information and the initial scanning data.

In one of the embodiments, the initial scanning data comprises multiple subsets of the initial scanning data collected by a medical scanning device at multiple scanning angles during the rotational scanning process, respectively, and dimension downsampling is performed on the initial scanning data to obtain intermediate scanning data, including:for one subset of the initial scanning data corresponding to each of the multiple scanning angles, down-sampling a first portion of the subset of the initial scanning data to obtain down-sampled merged data, wherein the subset of the initial scanning data includes the first portion and a second portion; andcombining the down-sampled merged data and the second portion as one subset of the intermediate scanning data corresponding to the each of the multiple scan angles.

In one of the embodiments, down-sampling the first portion of the subset of the initial scanning data to obtain the down-sampled merged data, including: dividing the first portion into one or more groups, wherein each of the one or more groups comprise a plurality of pixel values; summing the plurality of pixel values in each of the one or more groups to obtain the downsampled merged data.

In one of the embodiments, when dimension downsampling is performed along a channel direction of a detector of the medical scanning device, the first portion is a portion of the subset of the initial scanning data along the channel direction; or when dimensionally downsampling is performed along a row direction of the detector, the first portion is a portion of the initial scanning data along the row direction.

In one of the embodiments, the intermediate low-frequency information is an intermediate low-frequency image; extracting low-frequency information based on the intermediate scanning data to obtain the intermediate low-frequency information corresponding to the intermediate scanning data, including: reconstructing an intermediate reconstructed image based on the intermediate scanning data; extracting low-frequency information from the intermediate reconstructed image to obtain the intermediate low-frequency image corresponding to the intermediate scanning data.

In one of the embodiments, generating a target reconstruction image of the target object based on the intermediate low-frequency information and the initial scanning data, including: reconstructing an original reconstructed image based on the initial scanning data; extracting low-frequency information from the original reconstructed image to obtain an original low-frequency image corresponding to the initial scanning data; replacing the original low-frequency image in the original reconstructed image with the intermediate low-frequency image to obtain the target reconstruction image of the target object.

In one of the embodiments, if the size of the intermediate reconstructed image is smaller than that of the original reconstructed image, extracting low-frequency information from the intermediate reconstructed image to obtain the intermediate low-frequency image corresponding to the intermediate scanning data comprises: extracting low-frequency information from the intermediate reconstructed image to obtain a candidate low-frequency image; interpolating the candidate low-frequency image to obtain the intermediate low-frequency image corresponding to the intermediate scanning data.

In one of the embodiments, the intermediate low-frequency information is intermediate low-frequency data; extracting low-frequency information based on the intermediate scanning data to obtain the intermediate low-frequency information corresponding to the intermediate scanning data, including: extracting low-frequency information from the intermediate scanning data to obtain the intermediate low-frequency data corresponding to the intermediate scanning data.

In one of the embodiments, generating a target reconstruction image of the target object based on the intermediate low-frequency information and the initial scanning data, including: extracting low-frequency information from the initial scanning data to obtain original low-frequency data corresponding to the initial scanning data; replacing the original low-frequency data in the initial scanning data with the intermediate low-frequency data to obtain target scan data; reconstructing the target reconstruction image of the target object based on the target scanning data.

In one of the embodiments, the system comprising a detector array and a processor, wherein the detector array comprises multiple pixels along the channel direction and the row direction, and the multiple pixels are divided into multiple pixel blocks along the channel direction and/or the row direction, and each pixel block comprises multiple pixels; the pixel, configured to obtain the energy of the radiation beam while performing a CT scanning on the target object; the processor, configured to generate initial scanning data based on the radiation beam energy received by the detector array; wherein the initial scanning data includes data corresponding to each pixel; and to obtain intermediate scanning data based on initial scanning data; The intermediate scanning data includes data corresponding to each of the pixel blocks; and to extract low-frequency information based on the intermediate scanning data to obtain the intermediate low-frequency information corresponding to the intermediate scanning data; reconstructing a target reconstruction image of the target object based on the intermediate low-frequency information and the initial scanning data; the low-frequency information includes a low-frequency image or low-frequency data.

In one of the embodiments, wherein the data corresponding to the block pixel is obtained by summing data of each pixel corresponding to the block pixel in the initial scanning data.

In one of the embodiments, the pixel blocks are an array of pixels of $m \times n$; Wherein $m=1$ and $n \geq 2$; or $m \geq 2$ and $n = 1$; or, $m \geq 2$ and $n \geq 2$; $m$ is the number of pixels in the channel direction and $n$ is the number of pixels in the row direction; no overlapping pixels between each of the said pixel blocks, or overlapping pixels.

In one of the embodiments, the detector array comprises a photon counting detector or an energy integration detector.

In one of the embodiments, the initial scanning data comprises the initial scanning data of multiple scan angles collected by the CT during the rotation scanning, the system further comprises: the processor, configured to perform a summation process on multiple data within the pixel blocks of the original scan data for each scan angle, to obtain combined data; determining the scanning data of the original scan data which is in the non-pixel blocks, and combine the scan data in the non-pixel blocks with the scan data in the pixel blocks as the intermediate scan data corresponding to the scanning angle.

In one of the embodiments, one or more embodiments of the present disclosure provides a device comprising a processor. The processor is configured to implement a method for image reconstruction.

One or more embodiments of the present disclosure provides a computer-readable storage medium. The storage medium stores computer instructions, and when a computer reads the computer instructions from the storage medium, the computer executes a method for image reconstruction.

**BRIEF DESCRIPTION OF THE DRAWINGS**

The present disclosure will be further illustrated by way of exemplary embodiments, which will be described in detail using the accompanying drawings. These embodiments are not limiting, and in these embodiments, the same numbering denotes the same structure, wherein:

FIG. 1 is an environment diagram of the image reconstruction method in one embodiment;
FIG. 2 is a schematic diagram of the process of the image reconstruction method in one embodiment;
FIG. 3(a) is a schematic diagram of dimension downsampling for the channel direction in one embodiment;
FIG. 3(b) is a schematic diagram of dimension downsampling for the row direction in an embodiment;
FIG. 4 shows a flowchart of the image reconstruction method in another embodiment;
FIG. 5 is a flowchart of the image reconstruction method in another embodiment;
FIG. 6 shows a flowchart of the image reconstruction method in another embodiment;
FIG. 7 is a schematic diagram of the process for uniformity correction based on the image domain in one embodiment;
FIG. 8(a) is a schematic diagram of the original reconstructed image after conventional correction reconstruction in one embodiment;

FIG. 8(b) is a schematic diagram of the original low-frequency image after low-frequency filtering in one embodiment;

FIG. 9(a) is a schematic diagram of an intermediate reconstructed image after dimension downsampling in one embodiment;

FIG. 9(b) is a schematic diagram of an intermediate low-frequency image after dimensionality downsampling in an embodiment;

FIG. 10 is a schematic diagram of the target reconstruction image after low-frequency correction in one embodiment;

FIG. 11 is a schematic diagram of the process for uniformity correction based on the projection domain in an embodiment;

FIG. 12 is a block diagram of the image reconstruction device in one embodiment;

FIG. 13 shows an internal structure diagram of a computer device in an embodiment.

## DETAILED DESCRIPTION

To more clearly illustrate the technical solutions of the embodiments of the present disclosure, the accompanying drawings to be used in the description of the embodiments will be briefly described below. Obviously, the accompanying drawings in the following description are only some examples or embodiments of the present disclosure, and that the present disclosure may be applied to other similar scenarios in accordance with these drawings without creative labor for those of ordinary skill in the art. Unless obviously acquired from the context or the context illustrates otherwise, the same numeral in the drawings refers to the same structure or operation.

It should be understood that terms such as "system," "device," "equipment," "unit," and "module" as used herein is a way to distinguish between different components, elements, parts, sections, or assemblies at different levels. However, these words may be replaced by other expressions if they accomplish the same purpose.

As indicated in the present disclosure and in the claims, the singular forms "a," "an," and "the" may be intended to include the plural forms as well, unless the context clearly indicates otherwise. In general, the terms "comprise," "comprises," and/or "comprising," "include," "includes," and/or "including," when used in this disclosure, specify the presence of stated features, integers, steps, operations, elements, and/or components, but do not preclude the presence or addition of one or more other features, integers, steps, operations, elements, components, and/or groups thereof.

Flowcharts are used in the present disclosure to illustrate the operations performed by the system according to some embodiments of the present disclosure. It should be understood that the operations described herein are not necessarily executed in a specific order. Instead, they may be executed in reverse order or simultaneously. Additionally, one or more other operations may be added to these processes, or one or more operations may be removed.

During the CT scanning, when the signal received by the detector is very small or close to 0, it may cause deviations in the subsequent correction and reconstruction algorithms, resulting in low-frequency ring artifacts in the reconstructed image and affecting the uniformity of the CT image. Among the current correction methods for image inhomogeneity, adjacent channel interpolation is often used for prediction, or algorithms based on external information or physical models are used to predict and correct inhomogeneity. For the time being, there is no similar method of dimensionality downsampling and filtering low frequencies.

Moreover, the correction method of adjacent channel interpolation has a better interpolation effect, that is, a better uniformity correction effect, when the number of invalid channels corresponding to the unevenness is small; In cases where there are a large number of adjacent invalid channels corresponding to the non-uniformity, the interpolation effect of the invalid channels adjacent to the effective channels of the uniformity is better, while the interpolation effect of the invalid channels farther from the effective channels is worse, and there are still some artifacts in the reconstructed image, resulting in poor uniformity correction effect.

Correction methods based on external information or physical models require additional acquisition of external information or pre-acquisition of predictive physical models, and theoretical predictions are made through reference data or physical models to correct the unevenness, which involves a large amount of computation and low processing efficiency.

Based on this, an image reconstruction method is proposed in the present application, which performs dimension downsampling on the projection domain data to increase the statistics of the detector units after data merging, thereby ensuring the accuracy of the subsequent correction and reconstruction algorithms, and then performs low-frequency filtering on the merged data to replace the low-frequency components of the original data, ultimately achieving the purpose of correcting the uniformity of the image. To improve the uniformity of the reconstructed image.

In addition, the method of the present application is also applicable to CT device scanning with ordinary energy integration detectors and to the correction and reconstruction process of Photon Counting Computed Tomography (PCCT) images; Because in the low-dose, high-resolution mode of PCCT, the matter decomposition algorithm based on the maximum likelihood method at low counts cannot guarantee unbiasedness and may even lead to ray starvation, ultimately resulting in the inability to perform quantitative energy spectrum calculations in the high-resolution mode; When using the method of the present application for image reconstruction, the image artifacts caused by ray hunger can be solved through dimension downsampling and low-frequency filter replacement, thereby improving the uniformity of the image, solving the current problem of PCCT image reconstruction in the industry, and improving the image quality of PCCT in high-resolution mode.

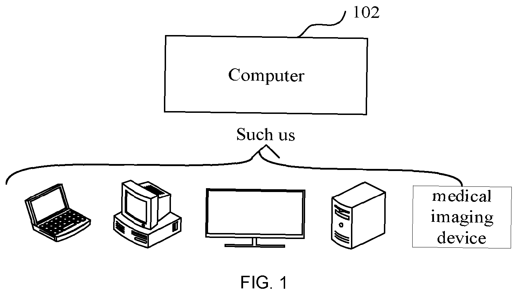

The image reconstruction method provided by the embodiments of the present application can be applied to the application environment shown in FIG. 1. Wherein the computer device 102 May include a medical imaging device or a reconstruction device communicating with the medical imaging device; For example, medical imaging equipment may include, but not be limited to, Computed Tomography (CT) equipment, Photon Counting Computed Tomography (PCCT) equipment, etc.

In addition, the reconstruction equipment may include but is not limited to reconstruction terminals and reconstruction servers, and the reconstruction terminals may but are not limited to personal computers, laptops, smart phones, tablets and portable wearable devices such as smartwatches, smart bands, headsets, etc. Reconstruction servers can be implemented using standalone servers or clusters of multiple servers, and can be local servers, remote servers, and cloud servers, etc.

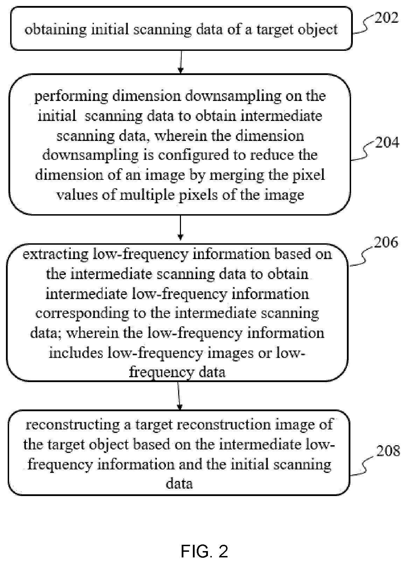

In an exemplary embodiment, as shown in FIG. 2, an image reconstruction method is provided, illustrated by the application of this method to the computer device in FIG. 1, including steps 202 to 206 below. Among them:

Step 202, obtaining initial scanning data of a target object.

The target object can be a living animal or human body, or a non-living model for research and development, testing, maintenance, and other scenarios. In addition, the target object can be the whole object or a local area of the object, such as a local tissue or organ. The type of target object is not specified in the embodiments of the present application.

For example, a medical scanning of the target object can be performed using a medical scanning device to obtain the initial scanning data of the target object; The raw scanning data can be the scanning data collected by the detector in the medical scanning device, which can also be called scan raw data. For example, computer devices can obtain raw scan data of the target object from medical scanning devices or from Picture Archiving and Communication systems (PACS), etc. In the embodiments of the present application, there is no specific limitation on the way the computer device acquires the initial scanning data of the target object.

Step 204: performing dimension downsampling on the initial scanning data to obtain intermediate scanning data, wherein the dimension downsampling is configured to reduce the dimension of an image by merging the pixel values of multiple pixels of the image.

wherein, dimension downsampling can include summation the data in the original scan that is within the preset merge range. Demonstratively, the preset merge range can be pre-determined based on the dimension downsampling magnification, for example: when the dimension downsampling magnification is 4, the preset merge range can include 2×2, 1×4, 4x1, etc.

The computer device can dimensionally downsample the initial scanning data according to the preset merge range by scanning row by row/column by summation the data within the preset merge range in the initial scanning data to obtain the dimensionally downsampled intermediate scanning data; In addition, multiple data within the preset merge range in the initial scanning data can be added to meet the statistical requirements. For example, if the initial scanning data is 4×12 scan data and the preset merge range is 2×2, that is, by statistically adding every 2×2 data in the 4×12 initial scanning data, you can obtain the dimensionally downsampled intermediate scanning data, that is, the intermediate scanning data is 2×6 scan data.

Step 206: extracting low-frequency information based on the intermediate scanning data to obtain intermediate low-frequency information corresponding to the intermediate scanning data; wherein the low-frequency information includes low-frequency images or low-frequency data.

Among them, the low-frequency information may include low-frequency images or low-frequency data; That is to say, the intermediate low-frequency information can be either an intermediate low-frequency image or an intermediate low-frequency data.

Low-frequency information usually refers to areas in an image where gray levels change slowly, and these areas typically appear as large color blocks or contiguous similar brightness .

For example, in the case of dimensionally downsampled intermediate scanning data, low-frequency information can be extracted from the intermediate scanning data to obtain the intermediate low-frequency data corresponding to the intermediate scanning data; Or, you can first perform preset data processing on the intermediate scanning data and then extract the low-frequency information from the processed intermediate scanning data to obtain the intermediate low-frequency information corresponding to the intermediate scanning data; Demonstratively, the preset data processing may include, but not be limited to, image reconstruction of the intermediate scanning data to obtain the reconstructed intermediate scanning image, and then extract the low-frequency information from the reconstructed intermediate scan image to obtain the intermediate low-frequency image.

Step 208: reconstructing a target reconstruction image of the target object based on the intermediate low-frequency information and the initial scanning data.

For example, when performing image reconstruction based on the intermediate low-frequency information and the initial scanning data, the original low-frequency information in the initial scanning data can be replaced based on the intermediate low-frequency information during the image reconstruction process to obtain the reconstructed target image with the replaced low-frequency information.

For example, computer devices can replace low-frequency information in the image domain and also in the projection domain; For example: When replacing low-frequency information in the image domain, the computer device can first reconstruct the image based on the dimensionally reduced intermediate scanning data to obtain the intermediate reconstructed image, then extract the intermediate low-frequency information from the intermediate reconstructed image and replace the original low-frequency information in the original reconstructed image based on the initial scanning data with this intermediate low-frequency information. To obtain the target reconstruction image of the target object.

For example, when replacing the low-frequency information in the projection domain, the computer device can first extract the intermediate low-frequency information from the dimensionally reduced intermediate scanning data, then replace the original low-frequency information in the initial scanning data based on this intermediate low-frequency information, and then perform image reconstruction based on the initial scanning data with the replaced low-frequency information, ultimately obtaining the target reconstruction image of the target object.

In the aforementioned image reconstruction method, the computer device acquires the initial scanning data of the target object and performs dimensionality downsampling on the initial scanning data to obtain the intermediate scanning data; Among them, dimension downsampling involves adding up the data within the preset merge range in the initial scanning data; Then extract the low-frequency information based on the intermediate scanning data to obtain the intermediate low-frequency information corresponding to the intermediate scanning data, and perform image reconstruction based on the intermediate low-frequency information and the initial scanning data to obtain the target reconstruction image of the target object; Here, the low-frequency information includes low-frequency images or low-frequency data. That is to say, in the image reconstruction method proposed in the embodiments of the present application, the statistics of the detector units after data merging are increased by dimensionally downsampling the data of the projection domain collected by the detector; That is, in the present application, the data of the unevenness is statistically fused to increase the statistics of the unevenness, and this statistics is obtained by merging the real collected data, rather than by interpolation prediction, or by prediction based on external reference information or physical model to increase the statistics of the unevenness; In other words, the method proposed in the present embodiment ensures the accuracy of subsequent correction and reconstruction by merging and downsampling the data collected by the detector without relying on external information such as interpolation prediction or model prediction, thereby improving the correction effect of the unevenness in the image and enhancing the uniformity of the image; In addition, the method proposed in the embodiments of the present application is universally applicable to image reconstruction in various scanning scenarios, with high universality and wide application range.

In an exemplary embodiment, the medical scanning device typically scans the target object when rotating to different scanning angles (views), for example: In the case of clockwise rotation, the medical scanning device can be respectively at 0° (the tube is vertically directly above and the detector is vertically directly below), 90° (the tube is horizontally on the right and the detector is horizontally on the left), 180° (the tube is vertically directly below and the detector is vertically directly above), and 270° (the tube is horizontally on the left) Scan at multiple angles such as the detector on the right side of the horizontal.

That is to say, the initial scanning data comprises multiple subsets of the initial scanning data collected by a medical scanning device at multiple scanning angles during the rotational scanning process; Then, when performing dimension downsampling in the projection domain, dimension downsampling of the initial scanning data for each scan angle is required view by view to obtain the intermediate scanning data for each scan angle. Based on this, the above step 204 can include: for one subset of the initial scanning data corresponding to each of the multiple scanning angles, down-sampling a first portion of the subset of the initial scanning data to obtain down-sampled merged data, wherein the subset of the initial scanning data includes the first portion and a second portion; and combining the down-sampled merged data and the second portion as one subset of the intermediate scanning data corresponding to the each of the multiple scan angles.

It should be noted that when dimensionally downsampling the initial scanning data for each scan angle, the preset merge range used can be the same, so that the initial scanning data for each scan Angle is subjected to the same dimensionally downsampling. For example, $4\times12$ of the initial scanning data for each scan Angle can be dimensionally downsampled according to the preset merge range of $2\times2$, resulting in $2\times6$ of the intermediate scanning data for each scan Angle.

Dimensionally, when dimensionally downsampling the initial scanning data for each scan angle, dimensionally downsampling can be performed on the initial scanning data for the scan angle as a whole, or dimensionally downsampling can be performed on the data of some regions in the initial scanning data, for example: dimensionally downsampling can be performed only on the non-uniform regions in the initial scanning data. Based on this, for each scan angle of the initial scanning data, the computer device can first determine the merged area of the initial scanning data, and then, in the merged area of the initial scanning data, add up multiple data within the preset merged range to obtain the merged data; And identify the scan data that the initial scanning data is in the non-merge area, and combine the scan data in the non-merge area with the merge data as the intermediate scanning data corresponding to the scan Angle.

Down-sampling the first portion of the subset of the initial scanning data to obtain the down-sampled merged data, including: dividing the first portion into one or more groups, wherein each of the one or more groups comprise a plurality of pixel values; summing the plurality of pixel values in each of the one or more groups to obtain the downsampled merged data.

That is to say, dimension downsampling can be performed on the data within the merged area of the initial scanning data, while dimension downsampling is not performed on the data within the non-merged area, that is, the unevenness part of the data can be statistically merged to increase the statistics of the unevenness part of the data.

For example, when dimension downsampling is performed along a channel direction of a detector of the medical scanning device, the first portion is a portion of the subset of the initial scanning data along the channel direction; , that is, after dimensionally downsampling along the channel direction, The length of the merged data corresponding to the row direction of the detector is equal to the length of the initial scanning data corresponding to the row direction.

When dimensionally downsampling is performed along a row direction of the detector, the first portion is a portion of the initial scanning data along the row direction.

In the case of dimension downsampling along the row direction of the detector, the merged area can be a part of the initial scanning data along the row direction, that is, after dimension downsampling along the row direction, the length of the merged data corresponding to the channel direction of the detector is equal to the length corresponding to the channel direction of the initial scanning data.

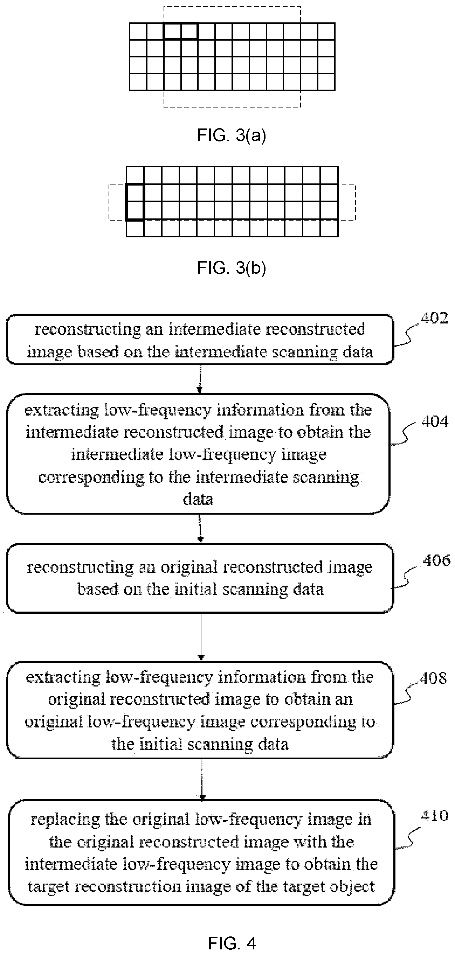

Based on the above example, assuming the initial scanning data is $4 \times 12$ scan data, when dimension reduction sampling is performed on the merged area in the channel direction, referring to FIG. 3(a), suppose dimension reduction sampling is performed on the data of the middle 8 channels in the channel direction (the merged area enclosed by the dotted line), then the preset merge range can be $1 \times n$, where, $2 \leq n \leq 8$; n is the number in the direction of the channel; In the case where the preset merge range is $1 \times 2$, that is, the statistical sum of every $1 \times 2$ data within the merge area (i.e., the data of the middle 8 channels) can result in the middle scan data of $4 \times 8$ after downsampling of the channel direction dimension; It is equivalent to compressing the initial scanning data in the channel direction dimension while keeping the dimension of the row direction of the initial scanning data unchanged.

Additionally, when dimension downsampling is performed on some of the merged regions in the row direction, as shown in FIG. 3(b), suppose dimension downsampling is performed on the data of the middle two channels in the row direction, then the preset merge range can be $m \times 1$, where $m=2$; m is the number in the row direction. In the case where the preset merge range is $2 \times 1$, that is, the statistical sum of every $2 \times 1$ data within the merge area (i.e., the data of the middle 2 rows) is performed, the middle scan data is $3 \times 12$ after downsampling of the row direction dimension; It is equivalent to compressing the dimension in the row direction of the initial scanning data while keeping the dimension in the channel direction of the initial scanning data unchanged.

In this example, for the initial scanning data collected at different scan angles during rotation, dimension downsampling is required separately, and when performing dimension downsampling, dimension downsampling can be performed on part or all of the area in the channel direction to achieve dimension compression in the channel direction; It is also possible to perform dimension reduction sampling on some or all of the areas in the row direction to achieve row direction dimension compression; Alternatively, you can perform overall dimension downsampling on the initial scanning data; Dimension downsampling is relatively flexible, and in practical applications, it can be processed flexibly according to actual needs.

In an exemplary embodiment, a low-frequency information replacement process based on the image domain is provided. When the intermediate low-frequency information is an intermediate low-frequency image, as shown in FIG. 4, the above step 206 may include steps 402 and 404. Wherein:

> Step 402: reconstructing an intermediate reconstructed image based on the intermediate scanning data.
> Step 404: extracting low-frequency information from the intermediate reconstructed image to obtain the intermediate low-frequency image corresponding to the intermediate scanning data.

The intermediate scanning data can include dimension downsampled intermediate scanning data corresponding to each scan angle.

For example, the computer device can perform image reconstruction based on the intermediate scanning data of each scan Angle separately to obtain intermediate reconstructed images of each scan Angle; Then, using the method of low-frequency filtering, low-frequency information is extracted from the intermediate reconstructed images of each scan angle respectively to obtain intermediate low-frequency images containing low-frequency information of each scan Angle; Among them, there are many low-frequency filtering methods. For example, two-dimensional Gaussian filtering can be selected for the image domain to extract low-frequency information from the intermediate scan image. In the embodiment of the present application, the low-frequency filtering processing method for the image domain is not specifically limited, nor is the cut-off frequency of the low-frequency filtering specifically limited. In practical applications, it can be flexibly set and adjusted according to actual needs.

Accordingly, continuing as shown in FIG. 4, the above step 208 May include steps 406 to 410, where:

> Step 406: reconstructing an original reconstructed image based on the initial scanning data.
> Step 408, extracting low-frequency information from the original reconstructed image to obtain an original low-frequency image corresponding to the initial scanning data.

For example, when replacing low-frequency information in the image domain, the computer device can also perform image reconstruction based on the initial scanning data, thereby obtaining the original reconstructed image corresponding to the initial scanning data; Then, low-frequency information at the same cut-off frequency is extracted from the original reconstructed image to obtain the original low-frequency image containing the low-frequency information.

For example, when performing image reconstruction, the initial scanning data can be corrected first, and then image reconstruction can be carried out based on the corrected initial scanning data; It should be noted that the correction here can be conventional projection data correction methods, including but not limited to air correction, attenuation correction, scattering correction, etc.

Of course, when performing image reconstruction on intermediate scanning data, it is also possible to first perform conventional correction on the intermediate scanning data, and then perform image reconstruction on the corrected intermediate scanning data to obtain the intermediate reconstruction image.

Step 410: replacing the original low-frequency image in the original reconstructed image with the intermediate low-frequency image to obtain the target reconstruction image of the target object.

For example, when a computer device extracts the original low-frequency image from the original reconstruction image and the intermediate low-frequency image from the intermediate reconstruction image, it can first perform image subtraction between the original reconstruction image and the original low-frequency image, that is, remove the low-frequency information from the original reconstruction image to obtain a reconstruction image that contains information other than the low-frequency information; Then, the reconstructed image, which contains information other than the low-frequency information, is fused with the intermediate low-frequency image to achieve the image processing effect of replacing the original low-frequency information in the original reconstructed image with the intermediate low-frequency information to obtain the target reconstruction image of the target object.

In this embodiment, for the low-frequency information of the merged data, it can be replaced in the image domain. Specifically, first perform image reconstruction based on the intermediate scanning data to obtain the intermediate reconstruction image, and extract the low-frequency information from the intermediate reconstruction image to obtain the intermediate low-frequency image corresponding to the intermediate scanning data; And, based on the initial scanning data, image reconstruction is performed to obtain the original reconstruction image, and low-frequency information is extracted from the original reconstruction image to obtain the original low-frequency image corresponding to the initial scanning data; Then, replace the original low-frequency image with an intermediate low-frequency image in the original reconstruction image to obtain the target reconstruction image of the target object. Using this method, it is possible to replace the low-frequency information in the original image in the image domain, thereby correcting the non-uniform low-frequency information in the image to improve the uniformity of the reconstructed image.

In an exemplary embodiment, it can be seen from the image domain-based low-frequency information replacement process shown in FIG. 4 above that in the process of low-frequency information replacement in the image domain, two image reconstruction processes need to be performed, namely image reconstruction of the initial scanning data to obtain the original reconstruction image, and image reconstruction of the intermediate scanning data to obtain the intermediate reconstruction image; Since the computational process of image reconstruction is complex and computationally intensive, in order to further improve the efficiency of image reconstruction, the intermediate reconstruction process can also be optimized in this embodiment to reduce the computational load of intermediate reconstruction.

For example, since the data volume of the intermediate scanning data is less than that of the initial scanning data, when performing image reconstruction based on the intermediate scanning data, it is possible to perform image reconstruction of the preset size based on the intermediate scanning data to obtain the intermediate reconstruction image. Here, the size of the intermediate reconstructed image is smaller than that of the original reconstructed image.

For example, to speed up the reconstruction, for the dimensionally downsampled intermediate scanning data, the reconstruction matrix can be reduced, resulting in an intermediate reconstruction image that is smaller than the original reconstruction image; for example, for a $512 \times 512$ original reconstruction image, a $128 \times 128$ matrix reconstruction can be performed on the dimensionally downsampled intermediate scanning data to obtain a $128 \times 128$ intermediate reconstruction image.

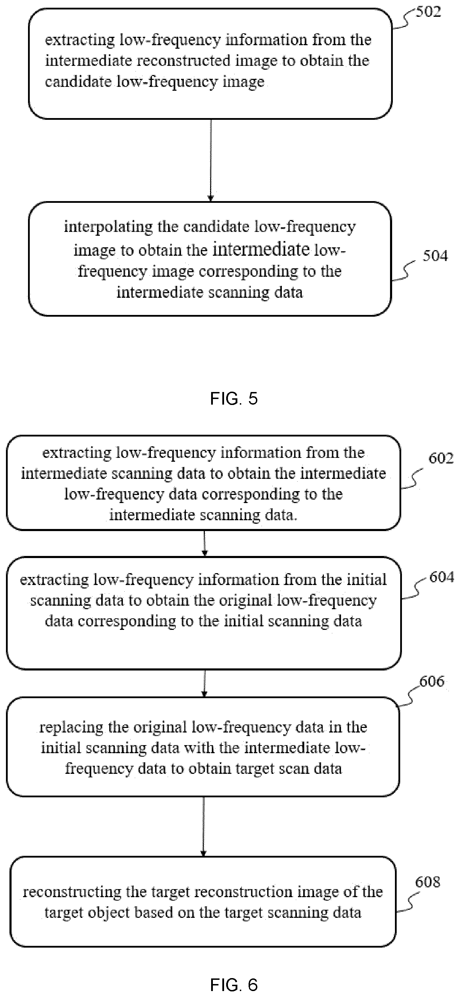

Based on this, as shown in FIG. 5, the above step 404 can include:

Step 502, extracting low-frequency information from the intermediate reconstructed image to obtain the candidate low-frequency image.

In the case, where the intermediate reconstructed image is a reconstructed image of the preset size, the candidate low-frequency image can be a low-frequency image of the preset size.

For example, in the case of obtaining an intermediate reconstruction image of a smaller size, low-frequency filtering can be used to extract low-frequency information from the intermediate reconstruction image, thereby obtaining a candidate low-frequency image of a preset size containing low-frequency information; For example: Get a 128×128 candidate low-frequency image containing low-frequency information.

Step 504, interpolating the candidate low-frequency image to obtain the intermediate low-frequency image corresponding to the intermediate scanning data.

Since the original reconstructed image is 512×512, the original low-frequency image extracted from the original reconstructed image will also be 512×512. When replacing the original low-frequency image in the original reconstructed image with an intermediate low-frequency image, it should be ensured that the size of the intermediate low-frequency image is consistent with that of the original low-frequency image. Therefore, in the case of obtaining a candidate low-frequency image of a smaller size, the candidate low-frequency image can be processed by interpolation to obtain an intermediate low-frequency image of the same size as the original low-frequency image.

In this embodiment, when replacing the low-frequency information in the image domain, in order to speed up the image reconstruction, for the dimensionally downsampled intermediate scanning data, the reconstruction matrix can be reduced to obtain a smaller-sized intermediate reconstruction image; Then, for the candidate low-frequency image extracted from the smaller-sized intermediate reconstruction image, an intermediate low-frequency image of the same size as the original reconstruction image can be obtained through interpolation, so that the low-frequency information in the original reconstruction image can be replaced later with an intermediate low-frequency image of the same size; While speeding up the reconstruction process, achieving image uniformity correction not only improves the efficiency of image reconstruction but also enhances the uniformity processing effect of image reconstruction.

In an exemplary embodiment, a low-frequency information replacement process based on the projection domain is provided. When the intermediate low-frequency information is intermediate low-frequency data, wherein the data includes projection data,as shown in FIG. 6, the above step 206 May include step 602. Wherein:

Step 602: extracting low-frequency information from the intermediate scanning data to obtain the intermediate low-frequency data corresponding to the intermediate scanning data.

For example, computer devices can extract low-frequency information directly in the data domain, that is, extracting low-frequency information from the intermediate scanning data, thereby obtaining intermediate low-frequency data in the intermediate scanning data. For example, a low-frequency filtering method for the data can be used to filter the intermediate scanning data to obtain the intermediate low-frequency data corresponding to the intermediate scanning data.

Accordingly, continuing as shown in FIG. 6, the above step 208 May include steps 604 to 608, wherein: Step 604, extracting low-frequency information from the initial scanning data to obtain original low-frequency data corresponding to the initial scanning data.

Similarly, the low-frequency filtering method for data in related techniques can also be used to perform low-frequency filtering on the initial scanning data to obtain the original low-frequency data corresponding to the initial scanning data. It should also be noted that when low-frequency filtering is applied to the intermediate scanning data and the initial scanning data, the low-frequency cut-off frequencies corresponding to the extracted low-frequency information should be consistent, that is, low-frequency information with the same cut-off frequency should be extracted to facilitate subsequent low-frequency information replacement.

Step 606: replacing the original low-frequency data in the initial scanning data with the intermediate low-frequency data to obtain target scan data.

For example, in cases where the initial scanning data includes multiple different scan angles of the initial scanning data, dimension downsampling can be performed separately for each scan angle of the initial scanning data to obtain the intermediate scanning data corresponding to each scan angle of the initial scanning data; Based on this, for each scan angle of the initial scanning data, use the intermediate low-frequency data extracted from the corresponding intermediate scanning data to replace the original low-frequency data in the corresponding initial scanning data, thereby obtaining the target scan data for each scan angle.

That is to say, in cases where there are multiple scan angles of the initial scanning data, the low-frequency information needs to be replaced separately for each scan angle of the initial scanning data, that is, corrected view by view, to ultimately obtain the target scan data corresponding to each scan Angle.

Step 608: reconstructing the target reconstruction image of the target object based on the target scanning data.

Here, the target scanning data includes target scan data from multiple scan angles.

For example, computer equipment can perform image reconstruction based on the target scanning data, thereby obtaining the corrected target reconstruction image. Among them, the method of image reconstruction may be a filtered backprojection reconstruction method or an iterative reconstruction method, etc., which is not specified in the present application embodiment.

In this embodiment, dimension downsampling is performed on the initial scanning data to obtain the intermediate scanning data, and low-frequency information is extracted from the intermediate scanning data to obtain the intermediate low-frequency data corresponding to the intermediate scanning data; And extract low-frequency information from the initial scanning data to obtain the original low-frequency data corresponding to the initial scanning data; Next, replace the original low-frequency data in the initial scanning data with the intermediate low-frequency data in the intermediate scanning data to obtain the target scan data; Then, based on the target scan data, image reconstruction is carried out to obtain the target reconstruction image of the target object. Using this method, it is possible to replace the low-frequency information in the initial scanning data in the projection domain, thereby correcting the unevenness of the data and improving the uniformity of the reconstructed image.

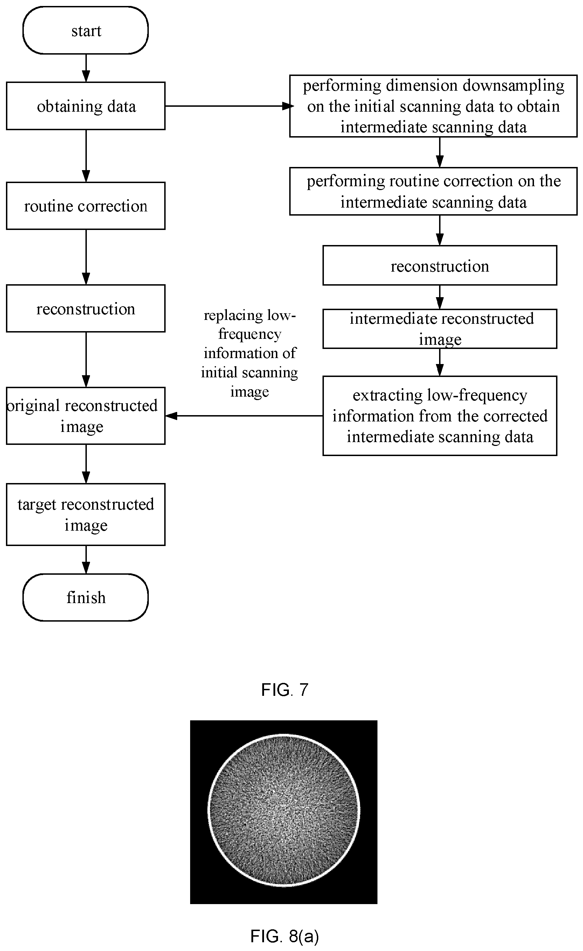

In an exemplary embodiment, as shown in FIG. 7, a reconstruction method for image uniformity correction based on the image domain is provided, the process of which includes the following steps:

Step 1: obtaining initial scanning data of the target object.

Step 2: performing routine reconstruction on the initial scanning data, that is, performing routine correction on the initial scanning data first, and then performing image reconstruction on the corrected initial scanning data to obtain the original reconstructed image, denoted as I1.

For example, test the water model, where the window width WW and window position WL of the reconstructed image are [WW, WL] = [50, 0]. Refer to FIG. 8(a), which shows the original reconstructed image I1 obtained after conventional processing.

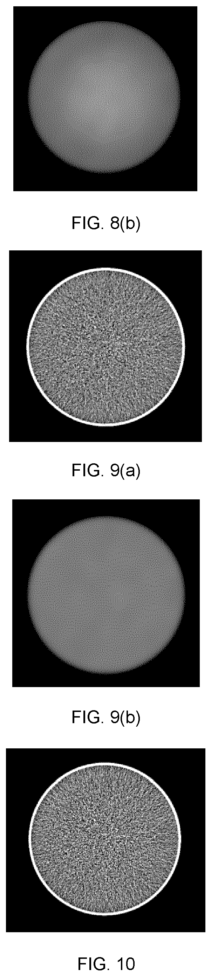

Step 3, performing low-frequency correction on the initial scanning data, that is, dimension downsampling of the initial scanning data first; for example, you can choose an appropriate preset merge range, such as a $2\times2$ merge range, to statistically sum every $2\times2$ in the initial scanning data to meet the statistical requirements and ensure that subsequent corrections and reconstructions are near the true value; By using the preset merge range, dimensionality downsampling of the initial scanning data is achieved to obtain intermediate scanning data; Then, regular correction and image reconstruction are performed on the dimensionally downsampled intermediate scanning data to obtain the dimensionally downsampled intermediate reconstructed image, denoted as I2. Refer to FIG. 9(a), which shows the intermediate reconstructed image I2 after low-frequency correction.

Step 4: extracting low-frequency information from the original reconstructed image I1 to obtain the original low-frequency image LP(I1), as shown in FIG. 8(b), and extracting low-frequency information at the same cut-off frequency from the dimensionally downsampled intermediate reconstructed image I2 to obtain the intermediate low-frequency image LP(I2), as shown in FIG. 9(b), and then, Replace the original low-frequency image LP(I2) in the original reconstructed image I2 with the intermediate low-frequency image LP(I1) based on the intermediate reconstructed image I1 to obtain the corrected target reconstructed image Iout, as shown in FIG. 10, which can be expressed as:

$$Iout = I1 - LP(I1) + LP(I2)$$

Where I1 is the original reconstructed image, I2 is the intermediate reconstructed image, LP(I1) is the original low-frequency image, LP(I2) is the intermediate low-frequency image, and Iout is the target reconstructed image.

By comparing FIG. 10 with FIG. 8(a), it can be seen that the signal in the central area of the original reconstructed image in FIG. 8(a) is weaker, the pixel distribution in the central area and the surrounding area of the reconstructed original reconstructed image is uneven, and the uniformity of the image is poor; By using the method proposed in the embodiments of the present application, after dimension downsampling and low-frequency information replacement of the initial scanning data, the pixels in the central area of the corrected target reconstruction image are consistent with those in the surrounding area, and the uniformity of the overall image is greatly improved.

Further, since image domain correction requires two reconstructs for each image, in order to accelerate the reconstruction, the reconstruction matrix can be reduced. In step 3 above, for the dimensionally downsampled intermediate scanning data, a smaller-sized matrix reconstruction can be performed on the intermediate scanning data at this time after conventional correction, The candidate low-frequency images obtained through filtering are then interpolated to obtain an intermediate low-frequency image of the same size as the original reconstructed image, so that the original low-frequency image in the original reconstructed image can be replaced later.

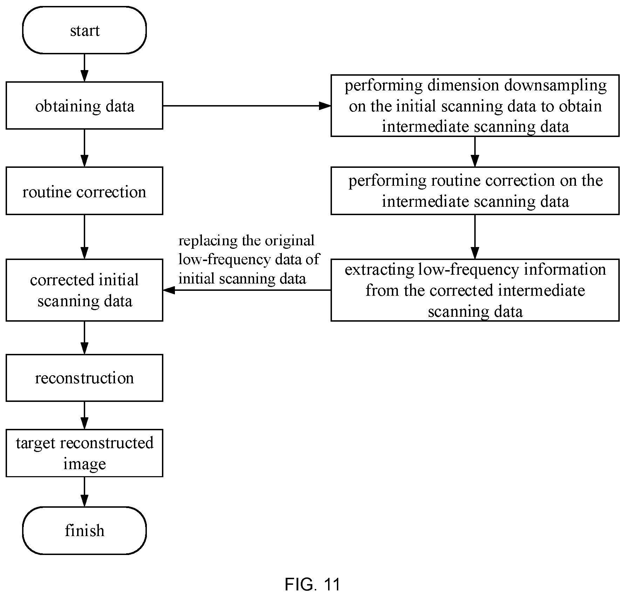

In an exemplary embodiment, as shown in FIG. 11, a reconstruction method for image uniformity correction based on the projection domain is provided, the process of which includes the following steps:

Step 1: obtaining the initial scanning data of the target object.

Step 2:performing routine correction processing on the initial scanning data to obtain the corrected initial scanning data.

Step 3: performing dimension downsampling on the initial scanning data to obtain the intermediate scanning data, and performing routine correction on the intermediate scanning data to obtain the corrected intermediate scanning data.

Step 4: extracting low-frequency information from the corrected intermediate scanning data to obtain the intermediate low-frequency data, and extract low-frequency information from the corrected initial scanning data to obtain the original low-frequency data. Using the intermediate low-frequency data extracted from the intermediate scanning data to replace the original low-frequency data extracted from the corresponding initial scanning data to obtain the target scan data.

Step 5: performing image reconstruction based on the target scan data to obtain the corrected target reconstruction image.

It should be noted that image domain correction is applied to the reconstructed single image, while projection domain correction requires individual correction of the initial scanning data at each scan Angle before reconstruction, followed by a reconstruction based on the corrected target scan data at each scan Angle to obtain the corrected target reconstruction image.

The image reconstruction method proposed in the present application, by merging and downsampling the initial scanning data collected by the detector in dimensions, improves the statistics of the detector units after data merging, does not rely on external information such as interpolation or model prediction, can ensure the accuracy of subsequent correction and reconstruction, eliminate low-frequency ring artifacts, and improve the uniformity of the image; In addition, by using this method and conducting dimensionally downsampling analysis on the initial scanning data, consistency between the low-frequency information and the low-frequency information structure of the original data can be guaranteed; And this method only changes the low-frequency components of the original image, without affecting the noise and resolution of the original image, thereby correcting the low-frequency artifacts and improving the quality of the reconstructed image. Furthermore, for the low-frequency information of the merged data after dimension downsampling, it can be replaced either in the projection domain or in the image domain; The method is not limited to the type of detector and can be used in traditional energy integration detector (EID) CT and photon counting detector (PCD) CT to address the ray starvation problem that may occur in PCCT in low-dose, high-resolution mode and improve the image quality of PCCT in high-resolution mode.

It should be understood that although the steps in the flowcharts of the embodiments mentioned above are shown in sequence as indicated by the arrows, these steps are not necessarily executed in the order indicated by the arrows. Unless explicitly stated herein, there are no strict sequence restrictions on the execution of these steps, and they may be executed in other sequences. Moreover, at least some of the steps involved in the flowcharts of the embodiments described above may include multiple steps or stages, which are not necessarily completed at the same time but can be executed at different times, and the order of execution of these steps or stages is not necessarily sequential. Instead, they may be executed alternately or in turn with other steps or at least part of steps or stages within other steps.

Based on the same inventive concept, the embodiments of the present application also provide an image reconstruction device for implementing the image reconstruction method involved above. The implementation of the solution provided by the device is similar to the implementation described in the above method. Therefore, the specific limitations in one or more of the image reconstruction device embodiments provided below can be seen in the above text for the limitations of the image reconstruction method and will not be elaborated here.

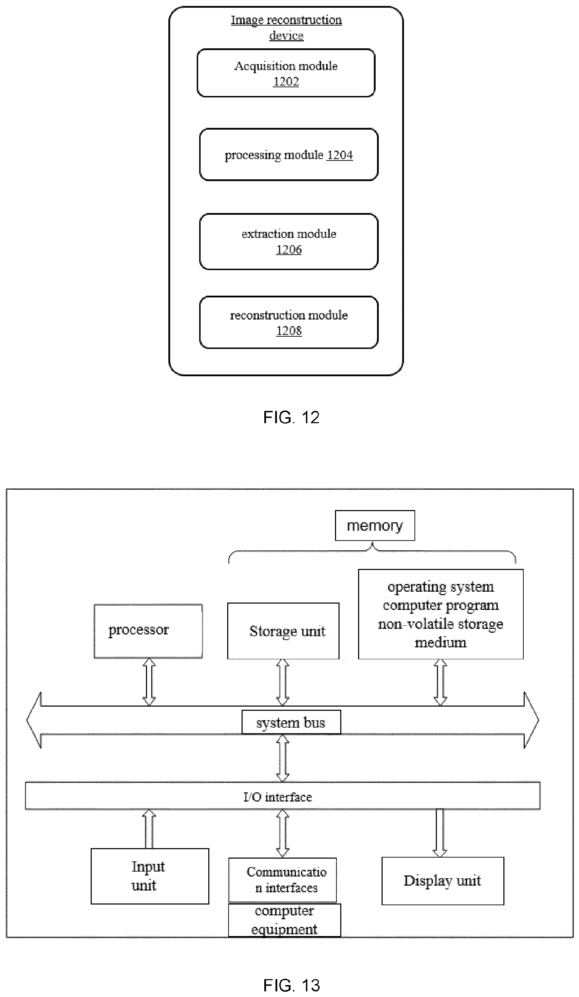

In an exemplary embodiment, as shown in FIG. 12, an image reconstruction device is provided, including: acquisition module 1202, processing module 1204, extraction module 1206 and reconstruction module 1208, wherein: the acquisition module 1202, configured to obtain the initial scanning data of the target object. processing module 1204, configured to perform dimension downsampling processing on the raw scan data to obtain intermediate scanning data; Dimension downsampling involves adding to the data within the preset merge range in the initial scanning data. extraction module 1206, configured to extract low-frequency information based on intermediate scanning data to obtain intermediate low-frequency information corresponding to intermediate scanning data; Low-frequency information includes low-frequency images or low-frequency data; reconstruction module 1208, configured to image reconstruction based on intermediate low-frequency information and raw scan data to obtain the target reconstruction image of the target object.

In one of the embodiments, the initial scanning data includes the initial scanning data of multiple scan angles collected by the medical scanning device during the rotation scan, and the intermediate scanning data includes the intermediate scanning data of each scan angle; processing module 1204, including:

processing units, configured to summing the initial scanning data within the preset merge range in the merge portion according to the initial scanning data for each scan Angle;

a determination unit, configured to determine the scan data that the initial scanning data is in the non-merged area and to combine the scan data in the non-merged area with the merged data as the intermediate scanning data corresponding to the scanning angle.

In one of the embodiments, where dimension downsampling is performed along the channel direction of the detector in the medical scanning device, the merged area is a portion of the initial scanning data along the channel direction; In the case of dimensionality reduction sampling along the row direction of the detector, the merged area is the portion of the initial scanning data along the row direction.

In one of the embodiments, the intermediate low-frequency information is a intermediate low-frequency image; extraction module 1206 comprises:

a first reconstruction unit is used for image reconstruction based on intermediate scanning data to obtain intermediate reconstructed images.

a first extraction unit is used to extract low-frequency information from the 2intermediate reconstructed image to obtain the intermediate low-frequency image corresponding to the intermediate scanning data.

In one of the embodiments, reconstruction module 1208 comprises:

a second reconstruction unit for image reconstruction based on the initial scanning data to obtain the original reconstructed image;

a second extraction unit is used to extract low-frequency information from the original reconstructed image to obtain the original low-frequency image corresponding to the initial scanning data;

a first replacement unit is used to replace the original low-frequency image with an intermediate low-frequency image in the original reconstruction to obtain the target reconstruction image of the target object.

In one of the embodiments, if the size of the intermediate reconstructed image is smaller than that of the original reconstructed image, the first extraction unit is used to extract low-frequency information from the intermediate reconstructed image to obtain the candidate low-frequency image; Interpolate the candidate low-frequency image to obtain the intermediate low-frequency image.

In one of the embodiments, the low-frequency information is low-frequency data; extraction module 1206 is used to extract low-frequency information from the intermediate scanning data to obtain the intermediate low-frequency data corresponding to the intermediate scanning data.

In one of the embodiments, reconstruction module 1208 comprises:

a third extraction unit for extracting low-frequency information from the initial scanning data to obtain the original low-frequency data corresponding to the initial scanning data;

a second replacement unit is used to replace the original low-frequency data in the initial scanning data with intermediate low-frequency data to obtain the target scan data;

a third reconstruction unit is used for image reconstruction based on the target scan data to obtain the target reconstruction image of the target object.

the modules in the aforementioned image reconstruction device can be implemented in whole or in part through software, hardware and their combinations. The modules may be embedded in or independent of the processor in the computer device in hardware form, or stored in the memory of the computer device in software form so that the processor can invoke and perform the operations corresponding to each module.

In an exemplary embodiment, a CT imaging system is provided, A CT imaging system, the system comprising a detector array and a processor, wherein the detector array comprises multiple pixels set along the channel direction and the row direction, and the multiple pixels are divided into multiple pixel blocks along the channel direction and/or the row direction, and each pixel block comprises multiple pixels;

the pixel, configured to obtain the energy of the radiation beam while performing a CT scanning on the target object;

the processor, configured to generate initial scanning data based on the radiation beam energy received by the detector array; wherein the initial scanning data includes data corresponding to each pixel; and to obtain intermediate scanning data based on initial scanning data; The intermediate scanning data includes data corresponding to each of the pixel blocks; and to extract low-frequency information based on the intermediate scanning data to obtain the intermediate low-frequency information corresponding to the intermediate scanning data; reconstructing a target reconstruction image of the target object based on the intermediate low-frequency information and the initial scanning data; the low-frequency information includes a low-frequency image or low-frequency data.the data corresponding to the block pixel is obtained by summing the data of each pixel corresponding to the pixel block in the initial scanning data.

The pixel blocks are an array of pixels of m×n; Wherein m=1 and n≥2; or m ≥2 and n = 1; or, m ≥2 and n≥2; m is the number of pixels in the channel direction and n is the number of pixels in the row direction; No overlapping pixels between each of the said pixel blocks, or overlapping pixels.

The detector array comprises a photon counting detector or an energy integration detector.

In an implementation, a CT imaging system may include an energy integration detector array and a processor; Among them, the energy integration detector array is composed of a scintillator, a photodiode array and a multi-channel analog-to-digital converter. The pixels of the energy integration detector are composed of detection units separated by physical partitions. Multiple pixels form an M×N detector array along the channel direction and the row direction, where M can be the number of pixels in the channel direction. That is, the number of pixels along the circumferential direction of the CT equipment frame; N can be the number of pixels in the row direction, that is, the number of pixels along the axial direction of the CT equipment frame.

Multiple pixels in the detector array can be divided into multiple pixel blocks along the channel direction and/or the row direction, that is, each pixel block contains multiple pixels. For example, for each pixel block, the pixel block can be an m×n pixel array; Among them, m represents the number of pixels in the channel direction of the pixel block, and n represents the number of pixels in the row direction of the pixel block.

> the pixel, configured to obtain the energy of the radiation beam while performing a CT scanning on the target object;
>
> the processor, configured to generate initial scanning data based on the radiation beam energy received by the detector array; wherein the initial scanning data includes data corresponding to each pixel; and to obtain intermediate scanning data based on initial scanning data; The intermediate scanning data includes data corresponding to each of the pixel blocks; and to extract low-frequency information based on the intermediate scanning data to obtain the intermediate low-frequency information corresponding to the intermediate scanning data; reconstructing a target reconstruction image of the target object based on the intermediate low-frequency information and the initial scanning data; the low-frequency information includes a low-frequency image or low-frequency data.

The energy integration detector uses a scintillation crystal to convert X-rays into visible light, and then a photodiode array converts the optical signal into an electrical signal. Relying on the cumulative measurement of X-ray photons by the energy integration detector, the pixel values obtained by the energy integration detector are the sum of the obtained energies, which are processed by adding the values of the total energy recorded by the detector.

In this example, the medical imaging device could be a PCCT device, that is, a photon-counting CT device; The PCCT device is equipped with a CT imaging system, which may include, but is not limited to, an array of photon counting detectors, processing circuits and reconstruction systems; Among them, the photon counting detector array is composed of multiple pixels that form an M×N detector array along the channel direction and the row direction, where M can be the number of pixels along the channel direction, that is, the number of pixels along the circumferential direction of the PCCT device rack; N can be the number of pixels in the row direction, that is, the number of pixels along the axial direction of the PCCT device rack.

For example, for multiple pixels in a photon counting detector array, multiple pixels can be divided into multiple pixels along the channel direction and/or row direction, that is, each pixel contains multiple pixels. For example, for each pixel, the pixel can be an m×n pixel array; Where m is the number of pixels in the channel direction and n is the number of pixels in the row direction of the pixel.

Demonstratively, m=1 and n≥2; Or, m≥2 and n = 1; Or, m≥2 and n≥2; That is, pixels can be row vector pixels, that is, pixels composed of multiple pixels in a single channel direction, that is, m=1 and n≥2; A pixel can also be a column vector pixel, that is, a pixel composed of multiple pixels in a single row direction, that is, m≥2 and n = 1; A pixel can also be a matrix pixel, that is, a pixel composed of multiple channel directions and multiple row directions, that is, m ≥2 and n≥2.

For example, for each pixel, there may be no overlapping pixels between the pixels, or there may be overlapping pixels. For an M×N detector array, each m×n pixel can be divided into a pixel if there is no overlap between the pixels; For example, a 4×12 detector array can be divided by 2×2 size, resulting in 12 pixels, that is, 2×6 pixels. If there are overlapping pixels among the pixels, then for an M×N detector array, the m×n detector array can be according to the size of M×N and the overlapping size of p×q to obtain multiple pixels; For example, for a 4×12 detector array, if the size is 4×2 and the overlap size is 4×1, you can get 4×11 pixels, that is, 44 pixels.

For each pixel, during subsequent data processing, the pixel value of all pixels I can summingto obtain the corresponding sum data of the pixel; The sum of the data corresponding to the multiple pixels can form the initial scanning data after dimension downsampling, that is, the intermediate scanning data.

It should be noted that for multiple pixels with overlapping pixels, the intermediate scanning data obtained after adding processing has less dimension reduction compared to the initial scanning data; For multiple pixels without overlapping pixels, the intermediate scanning data obtained after summing is significantly less dimensional compared to the initial scanning data. Compared with the image resolution obtained by correcting the image uniformity of the intermediate scanning data from multiple pixels without overlapping pixels, the image resolution obtained by correcting the image uniformity of the intermediate scanning data from multiple pixels with overlapping pixels is higher, that is, by using the overlapping ed partitioning method. It can improve the resolution of the image.

Then, in the case of adding the pixels to obtain the intermediate scanning data, the reconstruction system can extract the low-frequency information from that intermediate low-frequency information and replace the low-frequency information in the initial scanning data with the low-frequency information extracted from the intermediate low-frequency information during image reconstruction based on the initial scanning data. The uniform-corrected reconstructed image of the target.

The specific implementation process of image reconstruction based on low-frequency information can be described with reference to the relevant content of the image reconstruction method in any of the above embodiments, and will not be repeated here.

Photon counting detectors use semiconductor materials such as cadmium telluride (CdTe). When X-ray photons incident, their energy is absorbed by the semiconductor and converted into electrical signals. Each photon is directly counted and converted into a digital signal without the need for intermediate conversion steps such as photodiodes. Therefore, the pixel value obtained by the photon counting detector is the number of photons obtained, which is processed by summing the values of the number of photons recorded by the detector. The CT imaging system proposed in the embodiments of the present application can improve the uniformity correction effect when performing CT imaging, especially PCCT imaging, and obtain reconstructed images with higher uniformity, thereby improving the quality of reconstructed images.

In an exemplary embodiment, a computer device is provided, which can be a medical scanning device or a terminal or server communicating with the medical scanning device, and in the case where the computer device is a terminal, its internal structure diagram may be shown in FIG. 13. The computer device comprises a processor, memory, input/output interface, communication interface, display unit and input device. Among them, the processor, memory and input/output interfaces are connected via the system bus, and the communication interfaces, display units and input devices are connected to the system bus via the input/output interfaces. Among them, the processor of the computer device is used to provide computing and control capabilities. The memory of the computer device includes non-volatile storage media and internal memory. The non-volatile storage medium stores the operating system and computer programs. The internal memory provides the environment for the operation of the operating system and computer programs in the non-volatile storage medium. The input/output interface of the computer device is used to exchange information between the processor and external devices. The communication interface of the computer device is used for wired or wireless communication with external terminals. Wireless communication can be achieved via WIFI, mobile cellular networks, NFC (Near Field Communication), or other technologies. The computer program is executed by the processor to enable a method of image reconstruction. The display unit of the computer device is used to create a visually visible image, which can be a display screen, a projection device, or a virtual reality imaging device. The display can be a liquid crystal display or an e-ink display, and the input device of the computer device can be a touch layer covering the display, or a key, trackball or touchpad set on the computer device's casing, or an external keyboard, touchpad or mouse, etc.

It can be understood by those skilled in the art that the structure shown in FIG. 13 is merely a block diagram of a part of the structure related to the present application scheme and does not constitute a limitation to the computer equipment to which the present application scheme is applied. The specific computer equipment may include more or fewer components than shown in the figure or combine certain components, or have different component arrangements.

In an exemplary embodiment, a computer device is provided, including a memory and a processor, in which a computer program is stored and the processor performs the steps to implement the image reconstruction method in any of the above embodiments.

In one embodiment, a non-volatile computer-readable storage medium is provided on which a computer program is stored, and the steps for implementing the image reconstruction method in any of the embodiments are implemented when the computer program is executed by the processor.

In one embodiment, a product of a computer program is provided, including a computer program which, when executed by a processor, implements the steps of the image reconstruction method in any of the embodiments.

It should be noted that the data involved in the present application (including but not limited to data for analysis, data for storage, data for display, etc.) is information and data that have been fully authorized by all parties, and the collection, use and processing of the relevant data must comply with the relevant regulations.

It is understandable to those skilled in the art that the implementation of all or part of the processes in the embodiments above can be accomplished by instructing the relevant hardware through a computer program which can be stored in a non-volatile computer-readable storage medium and, when executed, may include processes such as those in the embodiments of the above methods. Any reference to memory, database or other medium used in the embodiments provided in the present application may include at least one of non-volatile and volatile memory. Non-volatile Memory may include Read-Only memory (read-only memory) ROM, magnetic tape, floppy disk, flash Memory, optical memory, high-density embedded non-volatile memory, Re-RAM, Magnetoresistive Random Access Memory MRAM, Ferroelectric Random Access Memory (FRAM), Phase Change Memory (PCM), graphene memory, etc. Volatile Memory can include Random Access Memory (RAM) or external cache memory, etc. As an illustration rather than a limitation, RAM can take various forms, such as Static Random Access Memory (SRAM) or Dynamic Random Access Memory (DRAM), etc. The databases involved in the embodiments provided in the present application may include at least one of a relational database and a non-relational database. Non-relational databases may include blockchain-based distributed databases, etc., but not limited to these. The processors involved in the embodiments provided in the present application may be general-purpose processors, central processing units, graphics processing units, digital signal processing units, programmable logic units, quantum computing-based data processing logic units, etc., not limited to these.

The technical features of the above embodiments can be combined in any way. For the sake of conciseness, not all possible combinations of the technical features of the above embodiments are described. However, as long as there is no contradiction in the combination of these technical features, they should be considered within the scope of this specification.

The embodiments described above only express several embodiments of the present application, which are described more specifically and in detail, but should not be construed as limiting the scope of the present application. It should be noted that for those skilled in the art, several variations and improvements can be made without departing from the conception of the present application, all of which fall within the scope of protection of the present application. Therefore, the scope of protection of the present application shall be based on the appended claims.

**Claims**

1. An method for image reconstruction, wherein the method comprises:

   obtaining initial scanning data of a target object;
   performing dimension downsampling on the initial scanning data to obtain intermediate scanning data; wherein the dimension downsampling is configured to reduce the dimension of an image by merging the pixel values of multiple pixels of the image;
   extracting low-frequency information based on the intermediate scanning data to obtain intermediate low-frequency information corresponding to the intermediate scanning data; wherein the low-frequency information includes low-frequency images or low-frequency data;
   reconstructing a target reconstruction image of the target object based on the intermediate low-frequency information and the initial scanning data.

2. The method of claim 1, wherein the initial scanning data comprises multiple subsets of the initial scanning data collected by a medical scanning device at multiple scanning angles during the rotational scanning process, respectively, and dimension downsampling is performed on the initial scanning data to obtain intermediate scanning data, including:

   for one subset of the initial scanning data corresponding to each of the multiple scanning angles,
   down-sampling a first portion of the subset of the initial scanning data to obtain down-sampled merged data, wherein the subset of the initial scanning data includes the first portion and a second portion; and
   combining the down-sampled merged data and the second portion as one subset of the intermediate scanning data corresponding to the each of the multiple scan angles.

3. The method of claim 2, wherein,
   down-sampling the first portion of the subset of the initial scanning data to obtain the down-sampled merged data, including:

   dividing the first portion into one or more groups, wherein each of the one or more groups comprise a plurality of pixel values;
   summing the plurality of pixel values in each of the one or more groups to obtain the down-sampled merged data.

4. The method of claim 2, wherein when dimension downsampling is performed along a channel direction of a detector of the medical scanning device, the first portion is a portion of the subset of the initial scanning data along the channel direction; or
   when dimensionally downsampling is performed along a row direction of the detector, the first portion is a portion of the initial scanning data along the row direction.

5. The method of any one of claims 1-4, wherein the intermediate low-frequency information is an intermediate low-frequency image; extracting low-frequency information based on the intermediate scanning data to obtain the intermediate low-frequency information corresponding to the intermediate scanning data, including:

   reconstructing an intermediate reconstructed image based on the intermediate scanning data;
   extracting low-frequency information from the intermediate reconstructed image to obtain the intermediate low-frequency image corresponding to the intermediate scanning data.

6. The method of claim 5, wherein generating a target reconstruction image of the target object based on the intermediate low-frequency information and the initial scanning data, including:

> reconstructing an original reconstructed image based on the initial scanning data;
> extracting low-frequency information from the original reconstructed image to obtain an original low-frequency image corresponding to the initial scanning data.
> replacing the original low-frequency image in the original reconstructed image with the intermediate low-frequency image to obtain the target reconstruction image of the target object.

7. The method of claim 6, wherein, if the size of the intermediate reconstructed image is smaller than that of the original reconstructed image, extracting low-frequency information from the intermediate reconstructed image to obtain the intermediate low-frequency image corresponding to the intermediate scanning data comprises:

> extracting low-frequency information from the intermediate reconstructed image to obtain a candidate low-frequency image;
> interpolating the candidate low-frequency image to obtain the intermediate low-frequency image corresponding to the intermediate scanning data.

8. The method of any one of claims 1-4, wherein the intermediate low-frequency information is intermediate low-frequency data; extracting low-frequency information based on the intermediate scanning data to obtain the intermediate low-frequency information corresponding to the intermediate scanning data, including:
extracting low-frequency information from the intermediate scanning data to obtain the intermediate low-frequency data corresponding to the intermediate scanning data.

9. The method of claim 8, wherein, reconstructing a target reconstruction image of the target object based on the intermediate low-frequency information and the initial scanning data, including:

> extracting low-frequency information from the initial scanning data to obtain original low-frequency data corresponding to the initial scanning data;
> replacing the original low-frequency data in the initial scanning data with the intermediate low-frequency data to obtain target scan data;
> reconstructing the target reconstruction image of the target object based on the target scanning data.

10. A CT imaging system, the system comprising a detector array and a processor, wherein the detector array comprises multiple pixels along the channel direction and the row direction, and the multiple pixels are divided into multiple pixel blocks along the channel direction and/or the row direction, and each pixel block comprises multiple pixels;

> the pixel, configured to obtain the energy of the radiation beam while performing a CT scanning on the target object;
> the processor, configured to generate initial scanning data based on the radiation beam energy received by the detector array; wherein the initial scanning data includes data corresponding to each pixel; and to obtain intermediate scanning data based on initial scanning data; The intermediate scanning data includes data corresponding to each of the pixel blocks; and to extract low-frequency information based on the intermediate scanning data to obtain the intermediate low-frequency information corresponding to the intermediate scanning data; reconstructing a target reconstruction image of the target object based on the intermediate low-frequency information and the initial scanning data; the low-frequency information includes a low-frequency image or low-frequency data.

11. The system of claim 10, wherein the data corresponding to the block pixel is obtained by summing data of each pixel corresponding to the block pixel in the initial scanning data.

12. The system of claim 10, wherein the pixel blocks are an array of pixels of m×n; Wherein m=1 and n≥2; or m≥2 and n = 1; or, m≥2 and n≥2; m is the number of pixels in the channel direction and n is the number of pixels in the row direction;
no overlapping pixels between each of the said pixel blocks, or overlapping pixels.

13. The system of claim 10, wherein the detector array comprises a photon counting detector or an energy integration detector.

14. The system of claim 10, wherein the initial scanning data comprises the initial scanning data of multiple scan angles collected by the CT during the rotation scanning, the system further comprises:
the processor, configured to perform a summation process on multiple data within the pixel blocks of the original scan data for each scan angle, to obtain combined data; determining the scanning data of the original scan data which is in the non-pixel blocks, and combine the scan data in the non-pixel blocks with the scan data in the pixel blocks as the intermediate scan data corresponding to the scanning angle.

**15.** A non-transitory computer-readable storage medium storing computer instructions, wherein when reading the computer instructions in the storage medium, a computer implements the method for image reconstruction of any of claims 1-9.

102

Computer

Such us

medical
imaging
device

FIG. 1

obtaining initial scanning data of a target object — 202

↓

performing dimension downsampling on the initial scanning data to obtain intermediate scanning data, wherein the dimension downsampling is configured to reduce the dimension of an image by merging the pixel values of multiple pixels of the image — 204

↓

extracting low-frequency information based on the intermediate scanning data to obtain intermediate low-frequency information corresponding to the intermediate scanning data; wherein the low-frequency information includes low-frequency images or low-frequency data — 206

↓

reconstructing a target reconstruction image of the target object based on the intermediate low-frequency information and the initial scanning data — 208

FIG. 2

FIG. 3(a)

FIG. 3(b)

reconstructing an intermediate reconstructed image based on the intermediate scanning data — 402

extracting low-frequency information from the intermediate reconstructed image to obtain the intermediate low-frequency image corresponding to the intermediate scanning data — 404

reconstructing an original reconstructed image based on the initial scanning data — 406

extracting low-frequency information from the original reconstructed image to obtain an original low-frequency image corresponding to the initial scanning data — 408

replacing the original low-frequency image in the original reconstructed image with the intermediate low-frequency image to obtain the target reconstruction image of the target object — 410

FIG. 4

extracting low-frequency information from the intermediate reconstructed image to obtain the candidate low-frequency image — 502

interpolating the candidate low-frequency image to obtain the intermediate low-frequency image corresponding to the intermediate scanning data — 504

FIG. 5

extracting low-frequency information from the intermediate scanning data to obtain the intermediate low-frequency data corresponding to the intermediate scanning data. — 602

extracting low-frequency information from the initial scanning data to obtain the original low-frequency data corresponding to the initial scanning data — 604

replacing the original low-frequency data in the initial scanning data with the intermediate low-frequency data to obtain target scan data — 606

reconstructing the target reconstruction image of the target object based on the target scanning data — 608

FIG. 6

start

obtaining data → performing dimension downsampling on the initial scanning data to obtain intermediate scanning data

routine correction

performing routine correction on the intermediate scanning data

reconstruction

reconstruction

original reconstructed image

replacing low-frequency information of initial scanning image

intermediate reconstructed image

extracting low-frequency information from the corrected intermediate scanning data

target reconstructed image

finish

FIG. 7

FIG. 8(a)

FIG. 8(b)

FIG. 9(a)

FIG. 9(b)

FIG. 10

```
         ┌──────────────┐
         │    start     │
         └──────┬───────┘
                │
                ▼
    ┌──────────────────┐        ┌────────────────────────────────┐
    │  obtaining data  │───────▶│ performing dimension downsampling│
    └──────────┬───────┘        │ on the initial scanning data to obtain│
               │                │   intermediate scanning data   │
               │                └───────────────┬────────────────┘
               ▼                                │
    ┌──────────────────┐                        ▼
    │ routine correction│        ┌────────────────────────────────┐
    └──────────┬───────┘        │ performing routine correction on the│
               │                │    intermediate scanning data   │
               │                └───────────────┬────────────────┘
               ▼          replacing the original │
    ┌──────────────────┐  low-frequency data of  ▼
    │ corrected initial │◀─initial scanning data ┌────────────────────────────────┐
    │  scanning data   │                        │ extracting low-frequency information│
    └──────────┬───────┘                        │  from the corrected intermediate│
               │                                │        scanning data           │
               ▼                                └────────────────────────────────┘
    ┌──────────────────┐
    │  reconstruction  │
    └──────────┬───────┘
               │
               ▼
    ┌──────────────────┐
    │ target reconstructed│
    │      image       │
    └──────────┬───────┘
               │
               ▼
         ┌──────────────┐
         │    finish    │
         └──────────────┘
```

FIG. 11

Image reconstruction
device

Acquisition module
1202

processing module 1204

extraction module
1206

reconstruction module
1208

FIG. 12

memory

operating system
computer program
non-volatile storage
medium

processor

Storage unit

system bus

I/O interface

Input
unit

Communicatio
n interfaces

Display unit

computer
equipment

FIG. 13

Europäisches
Patentamt
European
Patent Office
Office européen
des brevets

# EUROPEAN SEARCH REPORT

**Application Number**

EP 25 19 3733

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 117 152 288 A (NEUSOFT MEDICAL SYSTEMS CO LTD) 1 December 2023 (2023-12-01) | 1,5-13, 15 | INV. G06T11/00 |
| A | * the whole document * ----- | 2-4,14 | |
| A | US 2024/016459 A1 (ZHAN XIAOHUI [US] ET AL) 18 January 2024 (2024-01-18) * figures 3a, 3b, 4 * * paragraph [0065] - paragraph [0069] * ----- | 1-15 | |

**TECHNICAL FIELDS SEARCHED (IPC)**

G06T

The present search report has been drawn up for all claims

| Place of search | Date of completion of the search | Examiner |
|---|---|---|
| Munich | 22 December 2025 | Werling, Alexander |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.................................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 19 3733

This annex lists the patent family members relating to the patent documents cited in the above-mentioned European search report.
The members are as contained in the European Patent Office EDP file on
The European Patent Office is in no way liable for these particulars which are merely given for the purpose of information.

22-12-2025

| Patent document cited in search report | | Publication date | Patent family member(s) | | Publication date |
|---|---|---|---|---|---|
| CN 117152288 | A | 01-12-2023 | NONE | | |
| US 2024016459 | A1 | 18-01-2024 | JP | 2024010658 A | 24-01-2024 |
| | | | US | 2024016459 A1 | 18-01-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202411061950